(19) Europäisches Patentamt / European Patent Office / Office européen des brevets

(11) **EP 3 111 195 B1**

(12) **FASCICULE DE BREVET EUROPEEN**

(45) Date de publication et mention de la délivrance du brevet:
**24.05.2023 Bulletin 2023/21**

(21) Numéro de dépôt: **15707314.9**

(22) Date de dépôt: **23.02.2015**

(51) Classification Internationale des Brevets (IPC):
**G01N 21/47** *(2006.01)*    **G01N 15/02** *(2006.01)*
**G01N 33/483** *(2006.01)*    **C12M 1/34** *(2006.01)*

(52) Classification Coopérative des Brevets (CPC):
**G01N 21/4788; C12M 41/38; G01N 15/0211; G01N 33/4833**

(86) Numéro de dépôt international:
**PCT/EP2015/053676**

(87) Numéro de publication internationale:
**WO 2015/128271 (03.09.2015 Gazette 2015/35)**

(54) **PROCEDE ET DISPOSITIF POUR DETERMINER UNE CONCENTRATION EN LIPIDES DANS UNE MICRO ALGUE**

VERFAHREN UND VORRICHTUNG ZUR BESTIMMUNG EINER KONZENTRATION VON LIPIDEN IN EINER MIKROALGE

METHOD AND DEVICE FOR DETERMINING A CONCENTRATION OF LIPIDS IN A MICROALGAE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.02.2014 FR 1451495**

(43) Date de publication de la demande:
**04.01.2017 Bulletin 2017/01**

(73) Titulaire: **Commissariat à l'Énergie Atomique et aux Énergies Alternatives**
**75015 Paris (FR)**

(72) Inventeurs:
- **BOUTET, Jérôme**
  **F-38640 Claix (FR)**
- **ALLIER, Cédric**
  **F-38000 Grenoble (FR)**
- **FINAZZI, Giovanni**
  **F-38600 Fontaine (FR)**
- **MARECHAL, Eric**
  **F-38000 Grenoble (FR)**

(74) Mandataire: **Brevalex**
**56, Boulevard de l'Embouchure**
**B.P. 27519**
**31075 Toulouse Cedex 2 (FR)**

(56) Documents cités:
- **DAN FU ET AL: "Quantitative Chemical Imaging with Multiplex Stimulated Raman Scattering Microscopy", JOURNAL OF THE AMERICAN CHEMICAL SOCIETY, vol. 134, no. 8, 29 février 2012 (2012-02-29), pages 3623-3626, XP055133192, ISSN: 0002-7863, DOI: 10.1021/ja210081h**
- **GREENBAUM A ET AL: "Imaging without lenses: achievements and remaining challenges of wide-field on-chip microscopy", NATURE METHODS, NATURE PUBLISHING GROUP, GB, vol. 9, no. 9, 2012, pages 889-895, XP002698190, ISSN: 1548-7091, DOI: 10.1038/NMETH.2114 [extrait le 2012-08-30]**
- **C P Allier: "Bacteria detection with thin wetting film lensless imaging", Biomed. Opt. Express, 2010, pages 762-770, XP055066537, Extrait de l'Internet: URL:http://www.opticsinfobase.org/DirectPDFAccess/5DB6B7F5-D7B6-6C9A-B226E05846421E77_205470/boe-1-3-762.pdf?da=1&id=205470&seq=0&mobile=no [extrait le 2013-06-13]**

- **MONIKA E. DOLEGA ET AL: "Label-free analysis of prostate acini-like 3D structures by lensfree imaging", BIOSENSORS AND BIOELECTRONICS, vol. 49, 2013, pages 176-183, XP055132939, ISSN: 0956-5663, DOI: 10.1016/j.bios.2013.05.001**
- **J.-P Cadoret ET AL: "La production de biocarburant lipidique avec des microalgues : promesses et défis", Journal de la Societé de Biologie, 2008, pages 201-211, XP055133243, Extrait de l'Internet: URL:http://www-sop.inria.fr/comore/shamash /Cadoret_Bernard_BiodieselMicroalgues_2008 . pdf [extrait le 2014-08-05]**

**Description**

**DOMAINE TECHNIQUE**

La présente invention concerne le domaine des procédés pour déterminer une concentration en lipides dans une micro-algue.

L'invention concerne également un dispositif pour mettre en oeuvre un tel procédé.

**ÉTAT DE LA TECHNIQUE ANTÉRIEURE**

Une micro-algue est un organisme microscopique par exemple unicellulaire, et dont la taille varie typiquement de quelques micromètres à quelques centaines de micromètres. Les micro-algues regroupent typiquement des organismes photosynthétiques vivant essentiellement dans l'eau. Les micro-algues présentent un intérêt notamment dans le domaine des biocarburants, en raison de leurs propriétés de stockage des lipides, ces lipides pouvant ensuite servir à produire des carburants.

Par rapport aux sources de biocarburants traditionnelles, les micro-algues présentent de nombreux avantages, parmi lesquels une croissance très rapide de la biomasse, une faible consommation d'eau, l'absence de besoin d'une terre arable puisqu'elles sont cultivées en bassins ouverts ou en bioréacteurs. Elles offrent également un moyen avantageux de réutiliser le $CO_2$ produit par certaines industries. On estime à 200 000 le nombre d'espèce de micro-algues, dont seulement 35 000 ont été étudiées à ce jour. Il est donc très probable que l'on découvre rapidement des espèces de micro-algues particulièrement performantes dans le cadre de la production de biomasse, ce à quoi on doit ajouter la possibilité de créer de nouvelles variantes par des techniques de génie génétique.

La culture des micro-algues dans le cadre de la production des biomasses met en oeuvre une phase de stockage de lipides par les micro-algues, pour obtenir des micro-algues riches en lipides. Cette étape nécessite de connaître l'évolution de la concentration en lipides dans des micro-algues.

On connaît dans l'art antérieur un procédé de mesure de la concentration en lipides dans des micro-algues, consistant à prélever un échantillon de micro-algues que l'on met en contact avec un marqueur fluorescent nommé rouge de Nil. Le marqueur fluorescent se fixe d'autant plus aux micro-algues qu'elles sont riches en lipides, de sorte qu'on détecte un signal de fluorescence d'intensité proportionnelle à la concentration en lipides dans les micro-algues. Un inconvénient de ce procédé est qu'il est destructif pour l'échantillon, car le marqueur fluorescent est toxique pour les micro-algues. Il ne peut donc pas être mis en oeuvre *in situ,* dans un bioréacteur ou un bassin recevant une culture de micro-algues. Un tel procédé est décrit par exemple dans l'article « Nile red: a selective fluorescent stain for intracellular lipid droplets», P. Greenspan & al., The journal of Cell Biology, Vol. 100, mars 1985, pages 965-973.

Dans l'article « Quantitative Chemical Imaging with Multiplex Stimulated Raman Scattering Microscopy », Dan Fu & al., Journal of the American Chemical Society, vol. 134, no. 8, 29 février 2012, pages 3623-3626, les auteurs s'intéressent à la diffusion Raman stimulée, pour déterminer la composition d'un mélange ternaire.

Dans l'article « Imaging without lenses: achievements and remaining challenges of wide-field on-chip microscopy », Alon Greenbaum & al., Nature methods, Vol. 9, No. 9, septembre 2012, pages 889-895, les auteurs s'intéressent aux dispositifs d'imagerie sans lentille. Il n'est nullement question de la détermination d'une concentration en lipides.

Dans l'article « Bacteria detection with thin wetting film lensless imaging », C. P. Allier & al., Biomédical optics express, 1 octobre 2010, Vol. 1, No. 3, pages 762-770, il est décrit un dispositif d'imagerie sans lentille, pour compter et surveiller des micro-objets dans un échantillon liquide. Cet article ne s'intéresse pas à la détermination d'une concentration en lipides.

Dans l'article « Label-free analysis of prostate acini-like 3D structures by lensfree imaging », Monika E. Dolega & al., Biosensors and Bioelectronics, Vol. 49, 21 mai 2013, pages 176-183, les auteurs utilisent l'imagerie sans lentille pour discriminer rapidement des objets 3D de différentes polarités dans un large champ de vision afin de suivre la progression d'un cancer tel qu'un cancer de la prostate.

Un objectif de la présente invention est de proposer un procédé non destructif pour mesurer une concentration en lipides dans des micro-algues.

Un autre but de la présente invention est d'utiliser le procédé pour effectuer un suivi *in situ* d'une culture de micro-organismes dans un bassin ou un bioréacteur.

Un autre but de la présente invention est de proposer un dispositif pour mettre en oeuvre un tel procédé.

**EXPOSÉ DE L'INVENTION**

Cet objectif est atteint avec un procédé tel que défini dans la revendication indépendante 1 ci-jointe.

La dispersion de l'intensité lumineuse selon l'invention désigne une dispersion statistique.

Il s'agit de la mesure selon laquelle les valeurs de l'intensité lumineuse sont dispersées. La dispersion peut être mesurée par un écart type, un écart interquartile, une variance, une étendue, un écart moyen autour de la moyenne ou d'une autre valeur de référence, un diamètre d'ordre r et en particulier un diamètre d'ordre zéro, ou toute autre mesure connue d'une dispersion.

On peut définir une zone d'intérêt de façon à ce qu'elle comprenne une unique figure de diffraction élémentaire.

En variante, on peut définir une zone d'intérêt de façon à ce qu'elle comprenne une pluralité de figures de diffraction élémentaires, et on détermine une concentration moyenne en lipides dans les micro-algues d'intérêt.

De préférence, l'indicateur numérique consiste en un écart-type de l'intensité lumineuse relativement à une intensité lumineuse de référence.

En variante, l'indicateur numérique consiste, pour chaque figure de diffraction élémentaire, en une différence entre l'intensité lumineuse au centre de ladite figure de diffraction élémentaire et l'intensité lumineuse du premier anneau sombre de celle-ci.

Le procédé selon l'invention est avantageusement mis en oeuvre pour effectuer un suivi *in situ* d'une culture de micro-algues dans un bassin ou un bioréacteur, comme défini dans la revendication indépendante 6 ci-jointe.

L'invention concerne également un dispositif pour déterminer une concentration en lipides dans une micro-algue, tel que défini dans la revendication indépendante 7 ci-jointe.

Le dispositif selon l'invention peut comprendre :

- un premier logement étanche, recevant la source lumineuse et présentant une première fenêtre transparente entre la source lumineuse et le photodétecteur matriciel ;
- un second logement étanche, recevant le photodétecteur matriciel, et présentant une seconde fenêtre transparente entre la première fenêtre transparente et le photodétecteur matriciel ;

le premier logement étanche, la source lumineuse, le second logement étanche et le photodétecteur matriciel formant ensemble une sonde immergeable.

## BRÈVE DESCRIPTION DES DESSINS

La présente invention sera mieux comprise à la lecture de la description d'exemples de réalisation donnés à titre purement indicatif et nullement limitatif, en faisant référence aux dessins annexés sur lesquels :

- la figure 1 illustre de manière schématique un procédé selon l'invention ;
- les figures 2A et 2B illustrent des figures de diffraction globales selon l'invention, respectivement dans le cas de micro-algues riches en lipides et pauvres en lipides ;
- les figures 3A et 3B illustrent des zones d'intérêt selon l'invention, respectivement dans le cas de micro-algues riches en lipides et pauvres en lipides ;
- les figures 4A et 4B illustrent des figures de diffraction élémentaires selon l'invention, respectivement dans le cas de micro-algues riches en lipides et pauvres en lipides ;
- la figure 4C illustre des coupes des figures de diffraction élémentaires représentées aux figures 4A et 4B ;
- la figure 5 illustre de manière schématique un premier mode de réalisation d'un dispositif selon l'invention ; et
- la figure 6 illustre de manière schématique une utilisation d'un deuxième mode de réalisation de dispositif selon l'invention.

## EXPOSÉ DÉTAILLÉ DE MODES DE RÉALISATION PARTICULIERS

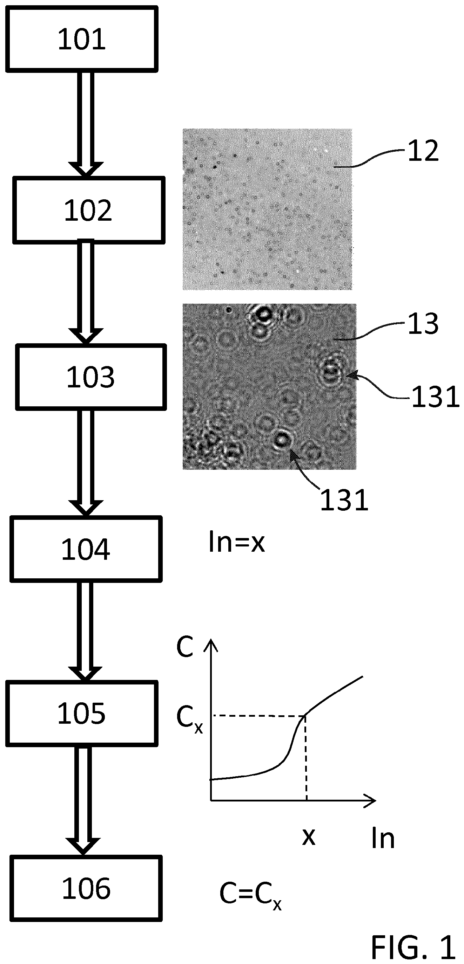

On va tout d'abord décrire, en référence à la figure 1, un procédé selon l'invention.

Etape 101 :

On illumine un échantillon contenant des micro-algues.

L'échantillon est par exemple une solution algale cultivée pendant plusieurs jours dans un milieu dit ESAW riche en azote. Le milieu ESAW correspond à une eau de mer artificielle, et il est décrit par Harrison et al. dans la revue « Journal of Phycology » vol. 16, pages 28-35, 1980 et, dans une version améliorée, par Berges et al. dans la revue « Journal of Phycology » vol. 37, pages 1138-1145, 2001. L'azote est essentiel pour la croissance algale. Les micro-algues sont en outre éclairées à 40 photons/micro-Einstein, 12 heures par jour et 12 heures par nuit, et soumises à une agitation continue. Les algues de la solution algale sont précisément du type *Phaeodactylum tricornutum* (Pt1) Bohlin Strain 8.6 CCMP2561 (disponibles dans la collection de la Marine Phytoplankton, nommé aujourd'hui NCMA pour « National Center for Marine Algae and Microbiota »). La concentration en micro-algues dans l'échantillon liquide est de $1.10^6$ à $2.10^6$ micro-algues/mL.

L'illumination correspond à une illumination par la source lumineuse de moyens d'imagerie sans lentille qui seront décrits plus avant dans la suite.

Etape 102 :

Lors de l'illumination 101 de l'échantillon, on acquiert une figure de diffraction globale, c'est-à-dire une image en deux dimensions comprenant une pluralité de figures de diffraction chacune associée à une micro-algue. Dans la suite, on nomme « figure de diffraction élémentaire » une figure de diffraction associée à une seule micro-algue.

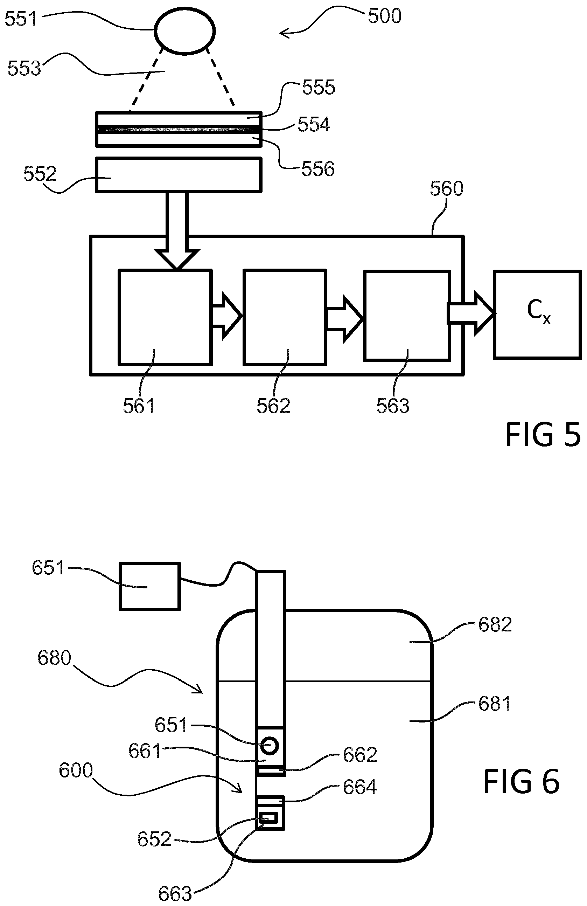

A titre d'illustration, on a représenté sur la droite un exemple d'une figure de diffraction globale 12. La figure de diffraction globale 12 correspond à une figure de diffraction obtenue à l'aide de moyens d'imagerie sans lentille tels qu'ils seront décrits dans la suite et en référence à la figure 5.

Etape 103 :

Au cours d'une étape 103, on définit, sur la figure de diffraction globale 12, une zone d'intérêt 13 représentée à droite sur la figure. La zone d'intérêt 13 comprend au moins une figure de diffraction élémentaire 131. Dans l'exemple représenté en figure 1, la zone d'intérêt 13 présente une pluralité de figures de diffraction élémentaires. On nomme « micro-algue d'intérêt » une micro-algue associée à une figure de diffraction élémentaire présente dans la zone d'intérêt 13, en particulier entièrement présente dans la zone d'intérêt, ou dont au moins la zone centrale et le premier anneau sombre sont présents dans la zone d'intérêt.

Comme explicité dans la suite, d'une façon générale, une figure de diffraction élémentaire comprend une zone centrale dont le niveau de gris est sensiblement homogène. Cette zone centrale est entourée par des anneaux concentriques, centrés sur la zone centrale. La zone centrale peut prendre la forme d'un disque, auquel cas les anneaux sont circulaires. Elle peut également prendre une autre forme, par exemple une surface en ellipse. Lorsque la zone centrale prend une forme de surface en ellipse, les anneaux ont une forme d'ellipse.

La sélection d'une zone d'intérêt 13 obéit de préférence à des critères préétablis. Par exemple, on sélectionne un rectangle présentant une hauteur et une largeur prédéfinies, et centré sur un pixel prédéterminé de l'image correspondant à la figure de diffraction globale. La zone d'intérêt est donc une image de taille inférieure ou égale à celle de la figure de diffraction globale 12.

On peut même envisager que la zone d'intérêt 13 présente les mêmes dimensions que la figure de diffraction globale 12. Dans ce cas, on peut s'affranchir de l'étape 103.

Etape 104 :

On détermine ensuite une valeur d'un indicateur numérique In, représentatif d'une dispersion de l'intensité lumineuse dans la zone d'intérêt 13.

L'indicateur numérique est par exemple un écart type de l'intensité lumineuse dans la zone d'intérêt 13, relativement à une intensité de référence qui peut être l'intensité lumineuse moyenne de cette zone d'intérêt 13. Il peut s'agir d'un écart-type relatif, c'est-à-dire un rapport entre l'écart-type et ladite intensité de référence.

En variante, l'indicateur numérique est la différence entre l'intensité lumineuse dans la zone centrale d'une figure de diffraction élémentaire 131 et l'intensité lumineuse dans le premier anneau sombre de cette figure de diffraction élémentaire 131.

Cette variante est particulièrement avantageuse dans le cas où la zone d'intérêt comprend une unique figure de diffraction élémentaire. En particulier :

- lorsque la zone d'intérêt comprend une unique figure de diffraction élémentaire 131, l'indicateur numérique peut être la différence entre l'intensité lumineuse dans la zone centrale et l'intensité lumineuse dans le premier anneau sombre de cette figure de diffraction élémentaire 131 ;
- lorsque la zone d'intérêt comprend plusieurs figures de diffraction élémentaires, l'indicateur numérique peut être une liste de différences entre l'intensité lumineuse dans la zone centrale et l'intensité lumineuse dans le premier anneau sombre de chaque figure de diffraction élémentaire, ou une valeur moyenne de ces différences.

On peut prendre en considération des valeurs moyennes d'intensité lumineuse dans ladite zone centrale et dans ledit premier anneau sombre. En d'autres termes, la différence entre l'intensité lumineuse dans la zone centrale et l'intensité lumineuse dans le premier anneau sombre d'une figure de diffraction élémentaire correspond alors à la différence entre l'intensité lumineuse moyenne dans la zone centrale et l'intensité lumineuse moyenne dans le premier anneau sombre de cette figure de diffraction élémentaire. En variante, cette différence correspond à la différence entre l'intensité lumineuse maximale dans la zone centrale et l'intensité lumineuse minimale dans le premier anneau sombre de cette figure de diffraction élémentaire.

Dans le cas où la zone d'intérêt comprend plusieurs figures de diffraction élémentaires, on peut considérer les figures de diffraction élémentaires séparément. L'indicateur numérique est alors une liste des différences entre l'intensité lumineuse dans la zone centrale et l'intensité lumineuse dans le premier anneau sombre, pour chaque figure de diffraction élémentaire de la zone d'intérêt. De préférence, l'indicateur numérique est cependant une valeur moyenne de ces différences.

Etape 105 :

On lit ensuite des données de calibration reliant une valeur de l'indicateur numérique et une concentration en lipides dans des micro-algues, pour relier la valeur calculée de l'indicateur numérique à la concentration en lipides dans les micro-algues de la zone d'intérêt précédemment sélectionnée. Connaissant la valeur prise par l'indicateur numérique, on en déduit, grâce aux données de calibration, une concentration en lipides $C_x$ dans les micro-algues d'intérêt.

Les données de calibration peuvent consister en une courbe reliant une valeur de l'indicateur numérique In et une concentration C en lipides dans les micro-algues.

En variante, on ne dispose pas d'une courbe mais de seulement un ou plusieurs points reliant une valeur de l'indicateur numérique In et une concentration C. Dans ce cas, la concentration en lipides dans les micro-algues d'intérêt est un intervalle de concentrations.

Les données de calibration sont typiquement déterminées lors d'une étape préalable de calibration, au cours de laquelle on mesure l'indicateur numérique correspondant à des micro-algues présentant une concentration connue en lipides. Au cours de cette étape de calibration, on acquiert par exemple plusieurs points reliant une valeur de l'indicateur numérique et une concentration en lipides, puis on en déduit une courbe par interpolation de ces points.

Cette étape de calibration doit être mise en oeuvre dans des conditions expérimentales prédéterminées et fixées, de façon à ce qu'entre deux mesures le contraste ne soit pas modifié autrement que par la solution algale elle-même. Ces conditions expérimentales sont notamment la nature, la position et la puissance d'émission d'une source lumineuse utilisée pour illuminer l'échantillon, et la nature et la position d'un capteur utilisé pour acquérir la figure de diffraction globale.

L'étape de calibration peut permettre de réunir des données de calibration utilisables quelles que soient les dimensions de la zone d'intérêt utilisée ensuite pour mettre en oeuvre le procédé selon l'invention. On voit par exemple que l'écart type d'une distribution d'intensités lumineuses dans une image ne dépend pas de la taille de cette image.

Il peut être avantageux de prendre en compte la concentration en micro-algues dans la solution algale. Dans ce cas, on effectue une calibration en deux dimensions en faisant varier à la fois la concentration en lipides des micro-algues et leur concentration dans la solution algale. En pratique, on disposera avantageusement d'une solution algale de concentration connue, ce qui permettra de déterminer une concentration en lipides en fonction d'une valeur de l'indicateur numérique et de cette concentration.

En fonction de la concentration en micro-algues dans la solution algale, on peut également déterminer des dimensions minimales pour la zone d'intérêt, permettant de s'assurer de moyenner un nombre suffisant de figures de diffraction élémentaires pour obtenir des résultats représentatifs.

Etape 106 :

L'étape 106 correspond à la fourniture de la concentration en lipides $C_x$ dans les micro-algues d'intérêt.

Il s'agit par exemple de la concentration moyenne en lipides dans les micro-algues d'intérêt, ou d'un intervalle de concentration en lipides dans les micro-algues d'intérêt.

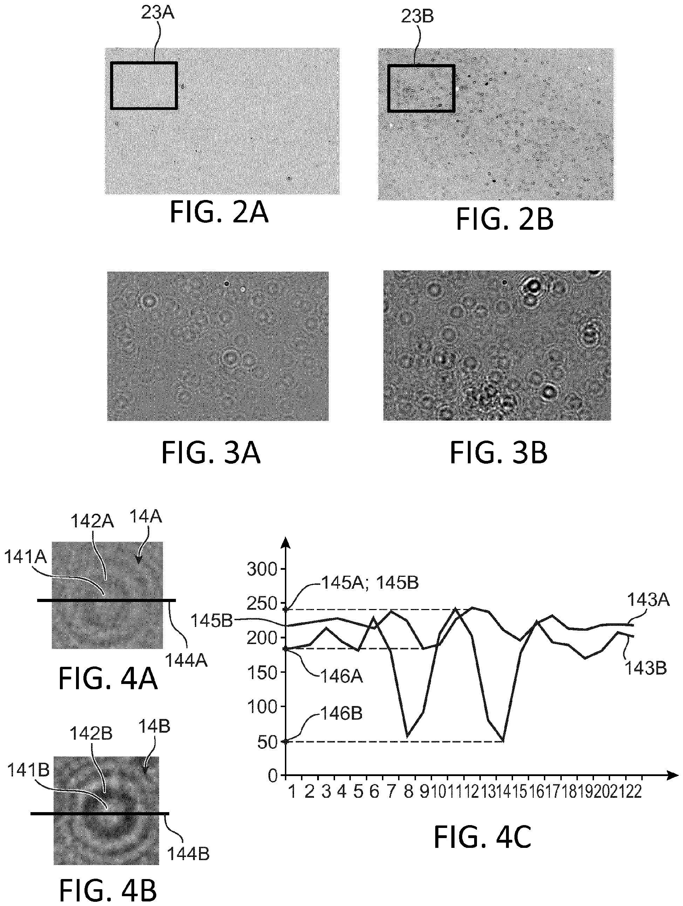

La figure 2A illustre une figure de diffraction globale d'un échantillon tel que défini en référence à la figure 1, et présentant une forte teneur en lipides. Cette forte teneur en lipides correspond ici à une teneur en triacylglycérol prenant une valeur comprise entre 20 et 80 % du poids sec. Le poids sec correspond à la masse des micro-algues après qu'elles ont été isolées de la solution (par exemple par centrifugation), puis lyophilisées.

L'échantillon correspondant a été obtenu à partir de la solution algale décrite ci-avant, et incubée ensuite pendant trois jours dans un milieu pauvre en nitrates (ici $NO_3^-$), donc pauvre en azote. En l'absence d'azote, les micro-algues ont tendance à grossir et à produire des lipides. Dans le cas présent, la concentration molaire en $NO_3^-$ est de 0 mol/L.

On a illustré en figure 2A une zone d'intérêt 23A telle que définie en référence à la figure 1, et comprenant plusieurs figures de diffraction élémentaires.

La figure 2B illustre une figure de diffraction globale d'un échantillon tel que défini en référence à la figure 1, et présentant une faible teneur en lipides. Cette faible teneur en lipides correspond ici à une teneur en triacylglycérol prenant une valeur comprise entre 0 et 10% du poids sec. L'échantillon correspondant a été obtenu à partir de la solution algale décrite ci-avant, et incubée ensuite pendant trois jours dans un milieu riche en nitrates (ici $NO_3^-$), donc riche en azote. En présence d'azote, les micro-algues ne produisent pas ou peu de lipides. Dans le cas présent, la concentration molaire en $NO_3^-$ est de 0,55 mmol/L.

On a illustré en figure 2B une zone d'intérêt 23B telle que définie en référence à la figure 1, et comprenant plusieurs figures de diffraction élémentaires.

Les figures 2A et 2B sont obtenues à l'aide d'un capteur CMOS de 24 mm² de surface, présentant un pas de pixel de 2,2 $\mu$m. On a placé sur le capteur une lame de microscope de 25×70 mm² de surface, sur laquelle on a déposé une goutte de la solution algale.

La figure 3A illustre la zone d'intérêt 23A représentée en figure 2A. La figure 3B illustre la zone d'intérêt 23B représentée en figure 2B. Sur ces figures, la largeur d'une figure de diffraction est d'environ 100 $\mu$m (diamètre du premier anneau sombre).

On voit sur les figures 3A et 3B que l'on peut reconnaître optiquement une zone d'intérêt correspondant à des micro-algues riches en lipides car les figures de diffraction élémentaires présentent un faible contraste. De la même manière, on peut reconnaître optiquement une zone d'intérêt correspondant à des micro-algues pauvres en lipides car les figures de diffraction élémentaires présentent un fort contraste.

On a mesuré, pour chaque zone d'intérêt, l'écart type de l'intensité lumineuse relativement à l'intensité lumineuse moyenne.

Dans le cas de la figure 3A, le signal présente un niveau de gris moyen de 216, un écart type de 14,0, soit un écart type relatif de 6,48%. Le niveau de gris sera défini en référence à la figure 4C.

Dans le cas de la figure 3B, le signal présente un niveau de gris moyen de 195, un écart type de 31,7, soit un écart type relatif de 16,3%.

Le rapport entre les deux écarts-types relatifs est suffisamment élevé (rapport 2,5) pour constituer un critère discriminant de la concentration en lipides dans une micro-algue.

On en déduit que :

- lorsque cet écart-type relatif est supérieur à 16,5%, la concentration en lipides dans une micro-algue est sensiblement inférieure à 10% du poids sec ;
- lorsque cet écart-type relatif est compris entre 6,5 et 16,5%, la concentration en lipides dans une micro-algue est sensiblement comprise entre 10% et 20% du poids sec ; et
- lorsque cet écart-type relatif est inférieur à 6,5%, la concentration en lipides dans une micro-algue est sensiblement supérieure à 20% du poids sec.

La figure 4A illustre une figure de diffraction élémentaire 14A associée à une micro-algue riche en lipides. Il s'agit d'une vue de détail de la figure 3A. On voit que la différence d'intensité lumineuse entre la zone centrale 141A et le premier anneau sombre 142A est faible.

La figure 4B illustre une figure de diffraction élémentaire 14B associée à une micro-algue pauvre en lipides. Il s'agit d'une vue de détail de la figure 3B. On voit que la différence d'intensité lumineuse entre la zone centrale 141B et le premier anneau sombre 142B est élevée.

On pourra donc déterminer une concentration en lipides dans une micro-algue à partir de sa figure de diffraction élémentaire, par exemple en déterminant l'intensité lumineuse moyenne dans la zone centrale, l'intensité lumineuse moyenne dans le premier anneau sombre, puis la différence entre les deux.

On peut considérer que les figures 4A et 4B correspondent chacune à une zone d'intérêt présentant une unique figure de diffraction élémentaire.

Ici, chaque micro-algue produit une figure de diffraction élémentaire comprenant un disque central clair (correspondant à une forte intensité lumineuse), entouré par une alternance d'anneaux sombres et clairs. Une modification de la géométrie d'une micro-algue (par exemple la géométrie de son enveloppe externe et/ou de son noyau), se traduira par une modification de la figure de diffraction élémentaire associée. On obtiendra par exemple une surface centrale ovale, entourée par des courbes fermées sombres et claires de forme ovale. La figure de diffraction élémentaire associée à une micro-algue présentera toujours une succession de courbes fermées concentriques, que par abus de langage on nommera également « anneaux de diffraction ». Ces courbes fermées entourent une surface nommée zone centrale.

Les lois de la diffraction permettent de prédire les dimensions de la zone centrale et celles du premier anneau sombre. On a en particulier pour une micro-algue présentant une géométrie à profil circulaire, une figure de diffraction élémentaire qui correspond à une tâche d'Airy. En particulier, le diamètre du plus petit anneau sombre est défini par la distance entre la micro-algue et le photodétecteur, et par un angle $\Theta$ tel que

$$\sin \theta = 1{,}22\frac{\lambda}{d}$$

, où $\lambda$ est la longueur d'onde d'émission de la source lumineuse, et $d$ est le diamètre du profil circulaire. On peut donc en déduire par exemple les dimensions d'une zone d'intérêt pour qu'elle comprenne une unique figure de diffraction élémentaire. En utilisant également un procédé de reconnaissance de forme, même très grossier, on pourra ensuite centrer la zone d'intérêt sur le centre d'une figure de diffraction élémentaire.

Puisque chaque figure de diffraction élémentaire présente ici une symétrie axiale, on peut travailler sur une coupe d'une figure de diffraction élémentaire passant par l'axe de symétrie. La figure 4C correspond à des coupes de deux figures de diffraction.

Sur la figure 4C, l'axe des ordonnées est gradué en niveau de gris (de 0 à 255, soient 256 niveaux de gris pour un signal analogique en sortie d'un photodétecteur converti en signal numérique codé sur 8 bits). Une faible valeur du niveau de gris correspond à une faible intensité lumineuse. Une forte valeur du niveau de gris correspond à une forte intensité lumineuse. L'axe des abscisses est gradué en pixels.

La courbe 143A correspond à une coupe de la figure de diffraction élémentaire représentée en figure 4A et passant par l'axe 144A coupant le centre de symétrie de la figure de diffraction élémentaire.

La courbe 143B correspond à une coupe de la figure de diffraction élémentaire représentée en figure 4B et passant par l'axe 144B coupant le centre de symétrie de la figure de diffraction élémentaire.

On reconnaît sur la figure 4C l'axe de symétrie de chaque figure de diffraction élémentaire. Les courbes 143A et 143B sont positionnées sur la figure de façon à ce que leurs axes de symétrie soient sensiblement alignés.

On peut distinguer en figure 4C :

- le maximum d'intensité 145A de la courbe 143A, situé dans la zone centrale 141A;
- le maximum d'intensité 145B de la courbe 143B, situé dans la zone centrale 141B (confondu ici avec le maximum d'intensité 145A) ;
- le minimum d'intensité 146A de la courbe 143A, situé dans le premier anneau sombre 142A; et
- le minimum d'intensité 146B de la courbe 143B, situé dans le premier anneau sombre 142B.

L'écart entre le maximum 145A et le minimum 146A vaut environ 50, alors que l'écart entre le maximum 145B et le minimum 146B vaut environ 200. Le rapport entre les deux écarts est suffisamment élevé (rapport 4) pour constituer un critère discriminant de la concentration en lipides dans une micro-algue.

On en déduit que :

- lorsque cet écart est supérieur à 200, la concentration en lipides dans une micro-algue est sensiblement inférieure à 10% du poids sec ;

- lorsque cet écart est compris entre 50 et 200, la concentration en lipides dans une micro-algue est sensiblement comprise entre 10% et 20% du poids sec ; et

- lorsque cet écart est inférieur à 50, la concentration en lipides dans une micro-algue est sensiblement supérieure à 20% du poids sec.

On voit donc que l'invention offre un procédé avantageux permettant de déterminer une concentration en lipides dans des micro-algues.

Ce procédé requiert une simple illumination d'un échantillon, et acquisition d'une figure de diffraction. Il est donc non destructif.

D'autre part, ce procédé permet d'analyser simultanément une pluralité de figures de diffraction élémentaires composant une figure de diffraction, chaque figure de diffraction élémentaire étant associée à une micro-algue.

Cela permet de caractériser, au même instant, la teneur lipidique d'un grand nombre de micro-algues, et de bénéficier d'indicateurs statistiques tels que la moyenne ou la variance. Par ailleurs, en reproduisant le procédé dans le temps, on obtient une information sur l'évolution temporelle de la teneur lipidique.

En outre, puisque l'on ne cherche pas ici à reconstruire l'image d'une micro-algue, il n'est pas nécessaire de connaître la distance entre la micro-algue et un capteur d'acquisition de la figure de diffraction globale. On peut donc facilement analyser une population de micro-algues contenues dans un volume, et pas seulement dans un plan. Il n'est pas non plus nécessaire de prévoir une focalisation sur un plan particulier, ce qui permet là-encore d'analyser facilement une population de micro-algues contenues dans un volume.

En outre, comme illustré dans la suite, les moyens matériels pour mettre en oeuvre ce procédé sont simples et peu onéreux : il s'agit en particulier de moyens d'imagerie sans lentille. Les moyens d'imagerie ne nécessitent pas l'utilisation d'optiques coûteuses. L'invention ne nécessite pas non plus l'utilisation de moyens de calcul très puissants, car on ne cherche pas à reconstruire une image des micro-algues à partir de leurs figures de diffraction élémentaires. On ne cherche pas non plus à traiter des figures de diffraction élémentaires pour les comparer à une bibliothèque de figures de diffraction de référence pour mettre ensuite en oeuvre des procédés lourds de reconnaissance de forme.

Puisque les calculs mis en oeuvre sont simples, le temps de traitement des informations peut être très court, inférieur à la seconde. Dans le cadre d'un suivi *in situ* d'une culture, cela permet de repérer rapidement une anomalie (due par exemple à un parasitage) et de procéder suffisamment tôt à la correction adéquate (par exemple par décontamination de la culture).

Un autre avantage du procédé selon l'invention est sa précision : on peut réaliser des mesures précises sur chaque micro-algue considérée individuellement, à partir de sa figure de diffraction élémentaire.

On va maintenant décrire en référence à la figure 5, un premier mode de réalisation d'un dispositif 500 selon l'invention. Le dispositif 500 selon l'invention est adapté à mettre en oeuvre le procédé selon l'invention.

Le dispositif 500 comprend en particulier des moyens d'imagerie sans lentille comprenant une source lumineuse 551 et un capteur 552. Les moyens d'imagerie sans lentille sont connus de l'homme de l'art, qui saura facilement trouver dans la littérature les détails de réalisation d'un tel dispositif.

Une source lumineuse 551 émet un faisceau lumineux 553 illuminant l'échantillon 554. La source lumineuse est, de préférence, cohérente spatialement. La source lumineuse peut être une diode laser, ou une diode électroluminescente (LED) suivie d'un trou de filtrage. Le trou de filtrage permet d'améliorer la cohérence spatiale du faisceau lumineux émis par la LED. Avantageusement, la source lumineuse est également temporellement cohérente.

On utilise par exemple une LED dont le spectre d'émission est centré sur 490 nm, soit une émission dans le bleu. La largeur du pic à 490 nm est de 40 nm. La puissance d'émission de la LED est par exemple comprise entre 100 mW et 1 W. La LED est suivie par un trou de filtrage de diamètre 150 $\mu$m, placé directement au contact de la LED.

L'échantillon 554 est placé entre la source lumineuse 551 et le capteur 552 consistant ici en un photodétecteur matriciel.

Dans l'exemple représenté sur la figure 5, l'échantillon est placé entre deux lamelles 555, 556 transparentes à la longueur d'onde d'émission de la source lumineuse. Le dispositif 500 selon l'invention travaille donc en transmission. La distance entre la lamelle 555 (du côté de la source lumineuse) et la source lumineuse est généralement comprise entre 1 cm et 10 cm.

L'échantillon est par exemple un échantillon d'une solution micro-algale telle que décrite en référence à la figure 1. Le dispositif 500 permet d'étudier des micro-algues contenues dans l'échantillon et présentant un faible diamètre, typiquement entre 100 nm et 500 $\mu$m, typiquement entre 50 $\mu$m et 200 $\mu$m.

Le photodétecteur matriciel 552 convertit un rayonnement électromagnétique incident en un signal électrique analogique. Ce photodétecteur matriciel 552 est généralement relié à un convertisseur analogique-numérique de façon à fournir une image numérique. On parle de photodétecteur matriciel, car la surface de détection du photodétecteur est découpée en pixels formant une matrice. Le photodétecteur matriciel 552 est par exemple un capteur CCD (pour l'anglais « *Charge-Coupled Device* ») ou un capteur CMOS (pour l'anglais «*Complementary Metal Oxide Semiconductor* »). Chaque pixel du photodétecteur est par exemple un carré de côté inférieur à 9 μm, et même inférieur à 5 μm, par exemple 2,2 μm. En particulier, on peut utiliser un capteur CMOS de 42,9×57,6 mm$^2$, présentant un pas de pixel de 2,2 μm.

Le photodétecteur matriciel 552 détecte une figure de diffraction correspondant à l'influence des micro-algues de l'échantillon 554 sur le faisceau lumineux 553. En particulier, le photodétecteur matriciel 552 détecte une figure de diffraction, correspondant aux interférences entre des ondes lumineuses incidentes provenant directement de la source lumineuse 551 et des ondes lumineuses émises par la source lumineuse puis diffractées par des micro-algues de l'échantillon 554. Cette figure de diffraction correspond à la figure de diffraction globale précédemment décrite. Chaque figure de diffraction élémentaire correspond donc aux interférences entre des ondes provenant directement de la source lumineuse 551 et des ondes émises par la source lumineuse puis diffractées par une micro-algue en particulier.

On nomme souvent « hologramme » une telle figure de diffraction. Un avantage d'une telle figure de diffraction est que le signal détecté est de grande amplitude, grâce à la contribution du signal provenant directement de la source lumineuse. Un autre avantage de cette figure de diffraction est que le champ de vision détecté est large car déterminé par la taille du capteur, par exemple supérieur à 20 mm$^2$. On peut ainsi imager simultanément des figures de diffraction élémentaires correspondant à de nombreuses micro-algues, par exemple jusqu'à dix mille.

Le photodétecteur 552 est positionné à proximité de l'échantillon 554, par exemple à 0,8 mm de la lamelle 556 (lamelle du côté du photodétecteur 552).

On détecte donc, à l'aide du dispositif 500 selon l'invention, une figure de diffraction correspondant à un objet, et non directement l'image de cet objet. On nomme cette technique « imagerie sans lentille ». Il faut noter que des moyens d'imagerie sans lentille peuvent comprendre une matrice de microlentilles, servant à focaliser sur chaque pixel le faisceau correspondant aux interférences à détecter. Cependant, ils ne comportent pas d'optique de grossissement disposée entre l'objet et le photodétecteur.

L'homme du métier saura aisément positionner les uns par rapport aux autres chacun des éléments parmi la source lumineuse 551, l'échantillon 554 et le photodétecteur 552.

On pourra prévoir un support (non représenté) pour recevoir l'échantillon 554, ce support étant disposé entre la source lumineuse 551 et le photodétecteur 552.

Dans l'exemple représenté, l'échantillon 554 est un échantillon volumique : l'épaisseur entre les deux lamelles 555, 556 est par exemple de 0,2 mm. Le volume d'échantillon 554, imagé sur le photodétecteur 552 sous la forme d'une figure de diffraction, est d'environ 5 μL.

Le dispositif 500 selon l'invention comprend des moyens de calcul 560 recevant en entrée la figure de diffraction globale acquise par le photodétecteur 552.

La figure de diffraction globale 500 est reçue par des moyens 561 pour sélectionner dans la figure de diffraction globale une zone d'intérêt telle que décrite en référence à la figure 1 et à propos de l'étape 103. Les moyens 561 comprennent avantageusement des moyens électroniques et des moyens informatiques et/ou logiciels, mettant en oeuvre un algorithme de sélection d'une partie d'une image en fonction de critères prédéfinis et contenus dans une mémoire tels que les dimensions de la partie d'image à sélectionner. On peut s'affranchir des moyens 561 dans le cas où la zone d'intérêt correspond à l'ensemble de la figure de diffraction globale.

L'image correspondant à cette zone d'intérêt est transmise à des moyens 562 pour déterminer une valeur d'un indicateur numérique représentatif d'une dispersion de l'intensité lumineuse dans la zone d'intérêt (voir étape 104 du procédé illustré en référence à la figure 1). Les moyens 562 comprennent avantageusement des moyens électroniques et des moyens informatiques et/ou logiciels, mettant en oeuvre un algorithme de mesure de niveaux de gris dans une image pour calculer un tel indicateur numérique.

La valeur ainsi calculée de l'indicateur numérique est transmise à des moyens 563 de lecture de données de calibration reliant une valeur d'un indicateur numérique et une concentration en lipides dans un micro-organisme d'intérêt, pour déterminer une concentration en lipides dans l'au moins un micro-organisme d'intérêt (voir étape 105 du procédé illustré en référence à la figure 1). Les moyens 563 comprennent avantageusement des moyens électroniques et des moyens informatiques et/ou logiciels, mettant en oeuvre :

- un algorithme de comparaison de données numériques (pour situer la valeur calculée de l'indicateur numérique sur l'axe des abscisses d'une courbe de calibration, ou dans un intervalle parmi plusieurs intervalles correspondant chacun à un intervalle entre les abscisses de deux points de calibration) ; et
- un algorithme de lecture de tables ou lecture de courbes (pour déterminer la concentration en lipides associée à une position sur l'axe des abscisses de la courbe de calibration ou un intervalle entre deux concentrations associé à un intervalle entre les abscisses de deux points de calibration).

La concentration en lipides $C_x$ ainsi obtenue est fournie en sortie par les moyens de calcul 560.

On voit que le dispositif selon l'invention est particulièrement simple, puisque les moyens d'imagerie sont minimaux et que les moyens de calculs ne requièrent pas une grande puissance de calcul, les traitements réalisés par ces derniers étant simples.

La figure 6 illustre une utilisation d'un deuxième mode de réalisation de dispositif selon l'invention. Sur la figure 6, les références numériques 600, 651, 652, 660 correspondent respectivement aux références numériques 500, 551, 552, 560 de la figure 5.

Le dispositif 600 selon l'invention comprend :

- un premier logement étanche 661 recevant la source lumineuse 651, et fermé par une première fenêtre transparente 662 ; et
- un deuxième logement étanche 663 recevant le photodétecteur 652, et fermé par une deuxième fenêtre transparente 664.

Le premier logement étanche 661 recevant la source lumineuse 651, et le deuxième logement étanche 663 recevant le photodétecteur 652, forment ensemble une sonde immergeable dans une solution liquide. L'échantillon est alors formé par la portion de solution liquide située entre la première et la deuxième fenêtres transparentes. Les fenêtres transparentes délimitent typiquement un volume de 5 μL, et sont espacées d'environ 200 μm l'une de l'autre. En variante, la distance entre les fenêtres transparentes peut être variable, de sorte que l'on peut ajuster cette distance pour obtenir le même nombre de micro-algues entre les fenêtres transparentes quelle que soit la concentration en micro-algues dans la solution liquide.

La sonde immergeable est reliée par voie filaire ou non filaire aux moyens de calcul 660.

Le dispositif 600 est placé dans un bioréacteur 680, notamment un photobioréacteur, c'est-à-dire un récipient fermé permettant de contrôler parfaitement les conditions de culture des micro-algues (température, pH, nutriments, $CO_2$). Un photobioréacteur présente un grand rapport surface/volume, ce qui limite l'hétérogénéité d'éclairage sur les micro-algues. Ce récipient fermé permet d'éviter la contamination des micro-algues par des parasites, et inversement, la contamination de l'environnement extérieur par les micro-algues.

Le bioréacteur 680 reçoit, dans une cuve 682, une solution liquide 681 comprenant des micro-algues. Des ouvertures pratiquées dans la cuve 682 permettent d'insérer des sondes pour contrôler les conditions à l'intérieur du bioréacteur 680. L'une de ces ouvertures est utilisée pour insérer la sonde immergeable telle que définie ci-dessus. La sonde immergeable présente une partie supérieure recevant le premier logement étanche tel que décrit ci-avant, et une partie inférieure recevant le second logement étanche tel que décrit ci-avant. L'espace entre ces deux logements est occupé par la solution liquide.

La sonde immergeable présente une forme de cylindre allongé, de diamètre inférieur à 12 mm et de longueur supérieure à 50 mm. Ses dimensions ne requièrent pas de modifier les bioréacteurs existants.

Le cylindre reçoit par exemple une carte électronique gérant l'acquisition des images par le photodétecteur et l'alimentation de la source lumineuse, cette carte étant pilotée à distance par un processeur recevant les moyens de calcul.

Le dispositif 600 selon l'invention permet de mesurer l'évolution au cours du temps d'une concentration en lipides dans les micro-algues de la solution liquide, par exemple pour déterminer le moment où la concentration en lipides est suffisante pour procéder à l'extraction des lipides. Le suivi *in situ* ne nécessite pas la présence d'un opérateur humain, puisque le procédé selon l'invention peut être automatisé.

Le temps d'acquisition d'une figure de diffraction globale est avantageusement de l'ordre de la milliseconde, pour éviter l'effet du mouvement des micro-algues.

En variante, on peut réaliser un suivi *in situ* dans un bassin ouvert utilisé pour la culture de micro-algues.

Dans cette divulgation, le terme "des micro-algues" signifie également des cyanobactéries.

Outre le domaine des biocarburants, l'étude de la concentration en lipides dans des micro-algues présente de nombreuses applications :

- dans le domaine agro-alimentaire, pour la fabrication de suppléments alimentaires ou la fabrication d'aliments pour les animaux (bétail, poissons) ;
- dans le domaine des biotechnologies, pour la production de molécules à haute valeur ajoutée, ou à des fins de criblage pour explorer la biodiversité ou isoler des mutants prometteurs à partir d'une banque de micro-algues.

**Revendications**

1. Procédé pour déterminer une concentration en lipides ($C_x$) dans une micro-algue, dans lequel :

   - on illumine (101) un échantillon (554) contenant des micro-algues;
   - on acquiert (102) une figure de diffraction globale (12) de l'échantillon, la figure de diffraction globale comprenant une pluralité de figures de diffraction élémentaires (131; 14A; 14B) associées chacune à une micro-algue et
   - on définit (103), sur la figure de diffraction globale (12), une zone d'intérêt (13, 23A, 23B) comprenant au moins une figure de diffraction élémentaire (131, 14A,14B), chaque figure de diffraction élémentaire de la zone d'intérêt étant associée à une micro-algue dit micro-algue d'intérêt; et chaque figure de diffraction élémentaire (131, 14A,14B) comprenant une zone centrale entourée par des anneaux concentriques, centrés sur la zone centrale;

   et comprenant les étapes suivantes :

   - détermination (104) d'une valeur d'un indicateur numérique (In) représentatif d'une dispersion de l'intensité lumineuse dans une zone d'intérêt (13 ; 23A ; 23B) de la figure de diffraction globale, l'indicateur numérique consistant soit en une différence soit en un écart-type entre des intensités lumineuses

- lecture (105) de données de calibration reliant la valeur de l' indicateur numérique (In) et une concentration (C) en lipides dans une micro-algue d'intérêt, pour déterminer la concentration en lipides ($C_x$) dans l'au moins une micro-algue d'intérêt, dans lequel

(i) lorsque l'indicateur numérique correspond à la différence entre l'intensité lumineuse dans la zone centrale d'une figure de diffraction élémentaire et l'intensité lumineuse dans le premier anneau sombre entourant la zone centrale et

- lorsque l'indicateur numérique est supérieur à 200, la concentration en lipides dans l'au moins une micro-algue d'intérêt est inférieure à 10% du poids sec ;
- lorsque l'indicateur numérique est compris entre 50 et 200, la concentration en lipides dans l'au moins une micro-algue d'intérêt est comprise entre 10% et 20% du poids sec; et
- lorsque l'indicateur numérique est inférieur à 50, la concentration en lipides dans l'au moins une micro-algue d'intérêt est supérieure à 20% du poids sec ;
(ii) lorsque l'indicateur numérique est l'écart-type relatif correspondant au rapport entre (a) l'écart-type de l'intensité lumineuse dans la zone d'intérêt relativement à l'intensité lumineuse moyenne dans cette zone d'intérêt et (b) cette intensité lumineuse moyenne,
- lorsque l'indicateur numérique est supérieur à 16,5%, la concentration en lipides dans l'au moins une micro-algue d'intérêt est inférieure à 10% du poids sec ;
- lorsque l'indicateur numérique est compris entre 6,5 et 16,5%, la concentration en lipides dans l'au moins une micro-algue d'intérêt est comprise entre 10% et 20% du poids sec ; et
- lorsque l'indicateur numérique est inférieur à 6,5%, la concentration en lipides dans l'au moins une micro-algue d'intérêt est supérieure à 20% du poids sec.

2. Procédé selon la revendication 1, dans lequel on définit une zone d'intérêt de façon à ce qu'elle comprenne une unique figure de diffraction élémentaire (14A ; 14B).

3. Procédé selon la revendication 1, dans lequel on définit une zone d'intérêt (13 ; 23A; 23B) de façon à ce qu'elle comprenne une pluralité de figures de diffraction élémentaires (131), et dans lequel on détermine une concentration moyenne en lipides dans les micro-algues d'intérêt.

4. Procédé selon l'une quelconque des revendications 1 à 3, dans lequel l'indicateur numérique (In) consiste en un écart-type de l'intensité lumineuse relativement à une intensité lumineuse de référence.

5. Procédé selon l'une quelconque des revendications 1 à 3, dans lequel l'indicateur numérique (In) consiste, pour chaque figure de diffraction élémentaire, en une différence entre l'intensité lumineuse (145A; 145B) au centre de ladite figure de diffraction élémentaire et l'intensité lumineuse (146A ; 146B) du premier anneau sombre de celle-ci.

6. Utilisation du procédé selon l'une quelconque des revendications 1 à 5, pour effectuer un suivi *in situ* d'une culture de micro-algues dans un bassin ou un bioréacteur (680).

7. Dispositif (500 ; 600) pour déterminer une concentration en lipides dans une micro-algue, en mettant en oeuvre le procédé selon l'une quelconque des revendications 1 à 5, le dispositif comprenant :

- une source lumineuse (551; 651), agencée pour illuminer un échantillon (554) comprenant des micro-algues ; et
- un photodétecteur matriciel (552 ; 652) disposé en face de la source lumineuse, agencé pour acquérir une image en deux dimensions représentant une figure de diffraction globale (12) de l'échantillon, la figure de diffraction globale comprenant plusieurs figures de diffraction élémentaires (131; 14A ; 14B) associées chacune à une micro-algue et des moyens de calcul (560 ; 660) recevant en entrée la figure de diffraction globale acquise par le photodétecteur matriciel (552; 652), lesdits moyens de calcul comprenant :

- des moyens (561) électroniques et des moyens informatiques et/ou logiciels agencé pour sélectionner dans la figure de diffraction globale une zone d'intérêt (13 ; 23A ; 23B) de la figure de diffraction globale associée à une micro-algue dit micro-algue d'intérêt;
- des moyens (562) électroniques et des moyens informatiques et/ou logiciels agencé pour déterminer une valeur d'un indicateur numérique représentatif d'une dispersion de l'intensité lumineuse dans la zone d'intérêt
- des moyens (563) électroniques et des moyens informatiques et/ou logiciels agencé pour la lecture de données de calibration reliant une valeur d'un indicateur numérique (In) et une concentration (C) en lipides dans une micro-algue d'intérêt.

8. Dispositif (600) selon la revendication 7, comprenant

- un premier logement étanche (661), recevant la source lumineuse (651) et présentant une première fenêtre transparente (662) entre la source lumineuse et le photodétecteur matriciel ;
- un second logement étanche (663), recevant le photodétecteur matriciel (652), et présentant une seconde fenêtre transparente (664) entre la première fenêtre transparente et le photodétecteur matriciel ;

le premier logement étanche, la source lumineuse, le second logement étanche et le photodétecteur matriciel formant ensemble une sonde immergeable.

**Patentansprüche**

1. Verfahren zur Bestimmung einer Lipidkonzentration ($C_x$) in einer Mikroalge, wobei:

- eine Probe (554), die Mikroalgen enthält, beleuchtet (101) wird;
- ein globales Beugungsmuster (12) der Probe erfasst (102) wird, wobei das globale Beugungsmuster eine Vielzahl elementarer Beugungsmuster (131; 14A; 14B) umfasst, die jeweils einer Mikroalge zugeordnet sind; und
- im globalen Beugungsmuster (12) ein Bereich von Interesse (13, 23A, 23B) definiert (103) wird, der mindestens ein elementares Beugungsmuster (131, 14A, 14B) umfasst, wobei jedes elementare Beugungsmuster des Bereichs von Interesse einer Mikroalge, Mikroalge von Interesse genannt, zugeordnet ist; und jedes elementare Beugungsmuster (131, 14A, 14B) einen zentralen Bereich umfasst, der von konzentrischen Ringen umgeben ist, die auf den zentralen Bereich zentriert sind;

und die folgenden Schritte umfassend:

- Bestimmung (104) eines Werts eines numerischen Indikators (In), der für eine Streuung der Lichtintensität in einem Bereich von Interesse (13; 23A; 23B) des globalen Beugungsmusters repräsentativ ist, wobei der numerische Indikator entweder aus einer Differenz oder einer Standardabweichung zwischen den Lichtintensitäten besteht;
- Ablesung (105) von Kalibrierungsdaten, die den Wert des numerischen Indikators (In) und eine Lipidkonzentration (C) in der Mikroalge von Interesse verknüpfen, um die Lipidkonzentration ($C_x$) in der mindestens einen Mikroalge von Interesse zu bestimmen, wobei,

(i) wenn der numerische Indikator der Differenz zwischen der Lichtintensität im zentralen Bereich eines elementaren Beugungsmusters und der Lichtintensität im ersten dunklen Ring entspricht, der den zentralen Bereich umgibt, und

- wenn der numerische Indikator größer als 200 ist, die Lipidkonzentration in mindestens einer Mikroalge von Interesse weniger als 10 % des Trockengewichts beträgt;
- wenn der numerische Indikator zwischen 50 und 200 liegt, die Lipidkonzentration in mindestens einer Mikroalge von Interesse zwischen 10 % und 20 % des Trockengewichts liegt; und,
- wenn der numerische Indikator weniger als 50 beträgt, die Lipidkonzentration in mindestens einer Mikroalge von Interesse mehr als 20 % des Trockengewichts beträgt;

(ii) wenn der numerische Indikator die relative Standardabweichung ist, die dem Verhältnis zwischen (a), der Standardabweichung der Lichtintensität im Bereich von Interesse relativ zur durchschnittlichen Lichtintensität in diesem Bereich von Interesse, und (b), dieser durchschnittlichen Lichtintensität entspricht,

- wenn der numerische Indikator größer als 16,5 % ist, die Lipidkonzentration in mindestens einer Mikroalge von Interesse weniger als 10 % des Trockengewichts beträgt;
- wenn der numerische Indikator zwischen 6,5 und 16,5 % liegt, die Lipidkonzentration in der mindestens einen Mikroalge von Interesse zwischen 10 % und 20 % des Trockengewichts liegt; und
- wenn der numerische Indikator weniger als 6,5 % beträgt, die Lipidkonzentration in mindestens einer Mikroalge von Interesse mehr als 20 % des Trockengewichts beträgt.

2. Verfahren nach Anspruch 1, wobei ein Bereich von Interesse so definiert wird, dass er ein einzelnes elementares Beugungsmuster (14A; 14B) umfasst.

3. Verfahren nach Anspruch 1, wobei der Bereich von Interesse (13; 23A; 23B) so definiert wird, dass er eine Vielzahl elementarer Beugungsmustern (131) umfasst, und wobei eine durchschnittliche Lipidkonzentration in den Mikroalgen von Interesse bestimmt wird.

4. Verfahren nach einem der Ansprüche 1 bis 3, wobei der numerische Indikator (In) aus einer Standardabweichung der Lichtintensität relativ zu einer Referenzlichtintensität besteht.

**5.** Verfahren nach einem der Ansprüche 1 bis 3, wobei der numerische Indikator (In), für jedes elementare Beugungsmuster, aus einer Differenz zwischen der Lichtintensität (145A; 145B) in der Mitte des elementaren Beugungsmusters und der Lichtintensität (146A; 146B) des ersten dunklen Rings davon besteht.

**6.** Verwendung des Verfahrens nach einem der Ansprüche 1 bis 5, um eine In-situ-Überwachung einer Mikroalgenkultur in einem Becken oder Bioreaktor (680) durchzuführen.

**7.** Vorrichtung (500; 600) zur Bestimmung einer Lipidkonzentration in einer Mikroalge durch Ausführung des Verfahrens nach einem der Ansprüche 1 bis 5, wobei die Vorrichtung umfasst:

- eine Lichtquelle (551; 651), die eingerichtet ist, um eine Probe (554) zu beleuchten, die Mikroalgen umfasst; und
- einen Matrix-Fotodetektor (552; 652), der gegenüber der Lichtquelle angeordnet ist, eingerichtet, um eine zweidimensionale Abbildung zu erfassen, die ein globales Beugungsmuster (12) der Probe darstellt, wobei das globale Beugungsmuster mehrere elementare Beugungsmuster (131; 14A; 14B) umfasst, die jeweils einer Mikroalge zugeordnet sind, und Berechnungsmittel (560; 660), die am Eingang das globale Beugungsmuster empfangen, das vom Matrix-Fotodetektor (552; 652) erfasst wurde,

wobei die Berechnungsmittel umfassen:

- elektronische Mittel (561) und Computerund/oder Softwaremittel, die eingerichtet sind, um aus dem globalen Beugungsmuster eines von Interesse (13; 23A; 23B) der globalen Beugungsmuster auszuwählen, das einer Mikroalge von Interesse zugeordnet ist;
- elektronische Mittel (562) und Computerund/oder Softwaremittel, die eingerichtet sind, um einen Wert eines numerischen Indikators zu bestimmen, der für eine Streuung der Lichtintensität im Bereich von Interesse repräsentativ ist;
- elektronische Mittel (563) und Computerund/oder Softwaremittel, die eingerichtet sind zur Ablesung von Kalibrierungsdaten, die einen Wert eines numerischen Indikators (In) und eine Lipidkonzentration (C) in einer Mikroalge von Interesse verknüpfen.

**8.** Vorrichtung (600) nach Anspruch 7, umfassend:

- ein erstes dichtes Gehäuse (661), das die Lichtquelle (651) aufnimmt und ein erstes transparentes Fenster (662) zwischen der Lichtquelle und dem Matrix-Fotodetektor aufweist;
- ein zweites dichtes Gehäuse (663), das den Matrix-Fotodetektor (652) aufnimmt und ein zweites transparentes Fenster (664) zwischen dem ersten transparenten Fenster und dem Matrix-Fotodetektor aufweist;

wobei das erste dichte Gehäuse, die Lichtquelle, das zweite dichte Gehäuse und der Matrix-Fotodetektor zusammen eine eintauchbare Sonde bilden.

**Claims**

**1.** Method for determining a lipid concentration ($C_x$) in a micro-alga, wherein:

- a sample (554) containing micro-algae is illuminated (101) ;
- an overall diffraction pattern (12) of the sample is acquired (102), the overall diffraction pattern comprising a plurality of elementary diffraction patterns (131; 14A; 14B) each associated with a micro-alga and
- an area of interest (13, 23A, 23B) comprising at least one elementary diffraction pattern (131, 14A, 14B) is defined (103) on the overall diffraction pattern (12), each elementary diffraction pattern of the area of interest being associated with a micro-alga called micro-alga of interest; and each elementary diffraction pattern (131, 14A, 14B) comprising a central area surrounded by concentric rings, which are centred on the central area; and comprising the following steps:

- determining (104) a value of a digital indicator (In) representative of a dispersion of the light intensity in an area of interest (13; 23A; 23B) of the overall diffraction pattern, the digital indicator consisting either in a difference or in a standard deviation between light intensities;
- reading (105) calibration data linking the value of the digital indicator (In) and a lipid concentration (C) in a micro-alga of interest, to determine the lipid concentration ($C_x$) in the au least one micro-alga of interest, in which

(i) when the digital indicator corresponds to the difference between the light intensity in the central area of an elementary diffraction pattern and the light intensity in the first dark ring surrounding the central area and

- when the digital indicator is greater than 200, the lipid concentration in the at least one micro-alga of interest is less than 10% of the dry weight;
- when the digital indicator is comprised between 50 and 200, the lipid concentration in the at least one micro-alga of interest is comprised between 10% and 20% of the dry weight; and
- when the digital indicator is less than 50, the lipid concentration in the at least one micro-alga of interest is greater than 20% of the dry weight;

(ii) when the digital indicator is the relative standard deviation corresponding to the ratio between (a) the standard deviation of the light intensity in the area of interest relative to the average light intensity in this area of interest and (b) this average light intensity,
- when the digital indicator is greater than 16.5%, the lipid concentration in the at least one micro-alga of interest is less than 10% of the dry weight;
- when the digital indicator is comprised between 6.5 and 16.5%, the lipid concentration in the at least one micro-alga of interest is comprised between 10% and 20% of the dry weight; and
- when the digital indicator is less than 6.5%, the lipid concentration in the at least one micro-alga of interest is greater than 20% of the dry weight.

2. Method according to claim 1, wherein an area of interest is defined so that it comprises a single elementary diffraction pattern (14A; 14B).

3. Method according to claim 1, wherein an area of interest (13; 23A; 23B) is defined so that it comprises a plurality of elementary diffraction patterns (131), and wherein an average lipid concentration is determined in the micro-algae of interest.

4. Method according to any one of claims 1 to 3, wherein the digital indicator (In) consists of a standard deviation of the light intensity relative to a reference light intensity.

5. Method according to any one of claims 1 to 3, wherein the digital indicator (In) consists, for each elementary diffraction pattern, of a difference between the light intensity (145A; 145B) at the centre of said elementary diffraction pattern and the light intensity (146A; 146B) of the first dark ring thereof.

6. Use of the method according to any one of claims 1 to 5, to perform an *in situ* monitoring of a micro-algae cultivation in a pond or a bioreactor (680).

7. Device (500; 600) for determining a lipid concentration in a micro-alga, by implementing the method according to any one of claims 1 to 5, the device comprising:

- a light source (551; 651), arranged to illuminate a sample (554) comprising micro-algae; and
- a matrix photodetector (552; 652) disposed facing the light source, arranged to acquire a two-dimensional image representing an overall diffraction pattern (12) of the sample, the overall diffraction pattern comprising several elementary diffraction patterns (131; 14A; 14B) each associated with a micro-alga, and
- calculation means (560; 660) receiving as input the overall diffraction pattern acquired by the matrix photodetector (552; 652), said calculation means comprising:

- electronic means (561) and computer and/or software means arranged to select, from the overall diffraction pattern, an area of interest (13; 23A; 23B) of the overall diffraction pattern associated with a micro-alga called micro-alga of interest;
- electronic means (562) and computer and/or software means arranged to determine a value of a digital indicator representative of a dispersion of the light intensity in the area of interest,
- electronic means (563) and computer and/or software means arranged to read calibration data linking a value of a digital indicator (In) and a lipid concentration (C) in a micro-alga of interest.

8. Device (600) according to claim 7, comprising:

- a first sealed housing (661), receiving the light source (651) and having a first transparent window (662) between the light source and the matrix photodetector;
- a second sealed housing (663), receiving the matrix photodetector (652), and having a second transparent window (664) between the first transparent window and the matrix photodetector;

the first sealed housing, the light source, the second sealed housing and the matrix photodetector together forming an immersible probe.

FIG. 1

FIG. 2A

FIG. 2B

FIG. 3A

FIG. 3B

FIG. 4A

FIG. 4B

FIG. 4C

FIG 5

FIG 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **P. GREENSPAN.** Nile red: a selective fluorescent stain for intracellular lipid droplets. *The journal of Cell Biology,* Mars 1985, vol. 100, 965-973
- **DAN FU.** Quantitative Chemical Imaging with Multiplex Stimulated Raman Scattering Microscopy. *Journal of the American Chemical Society,* 29 Février 2012, vol. 134 (8), 3623-3626
- **ALON GREENBAUM.** Imaging without lenses: achievements and remaining challenges of wide-field on-chip microscopy. *Nature methods,* Septembre 2012, vol. 9 (9), 889-895

- **C. P. ALLIER.** Bacteria detection with thin wetting film lensless imaging. *Biomédical optics express,* 01 Octobre 2010, vol. 1 (3), 762-770
- **MONIKA E. DOLEGA.** Label-free analysis of prostate acini-like 3D structures by lensfree imaging. *Biosensors and Bioelectronics,* 21 Mai 2013, vol. 49, 176-183
- **HARRISON et al.** *Journal of Phycology,* 1980, vol. 16, 28-35
- **BERGES et al.** *Journal of Phycology,* 2001, vol. 37, 1138-1145